# FASCICULE DE BREVET EUROPEEN

(11) **EP 3 218 273 B1**
(45) Date de publication et mention de la délivrance du brevet: **18.09.2019**
(21) Numéro de dépôt: 15797896.6
(22) Date de dépôt: 10.11.2015
(51) Int. Cl.: B65D 5/38, A01K 67/033, B65D 85/50, B65D 5/36

(54) **ETUI DE PROTECTION POUR UN CONDITIONNEMENT D'AUXILIAIRES POUR LA LUTTE BIOLOGIQUE**
SCHUTZHÜLLE FÜR EINE VERPACKUNG FÜR NÜTZLICHE ORGANISMEN ZUR BIOLOGISCHEN BEKÄMPFUNG
PROTECTIVE CASE FOR A PACKAGING FOR BENEFICIAL ORGANISMS FOR BIOLOGICAL CONTROL

(30) Priorité: 10.11.2014 FR 1460868
(43) Date de publication de la demande: 20.09.2017
(73) Titulaire: Bioline France, 75016 Paris (FR)
(72) Inventeur: FRANDON, Jacques, F-06600 Antibes (FR); TOURNIAIRE, Réjane, F-26270 Loriol Sur Drome (FR)
(74) Mandataire: Regimbeau
(86) Numéro de dépôt international: PCT/EP2015/076247
(87) Numéro de publication internationale: WO 2016/075160

(56) Documents cités:
- EP-A1- 2 380 437
- DE-U1- 9 306 953
- US-A- 2 403 840
- US-A- 3 111 223
- US-A1- 2005 120 963

## Description

La présente invention concerne un ensemble pour conditionner, protéger et libérer des insectes et/ou acariens auxiliaires dans le cadre de la lutte biologique, comme par exemple des trichogrammes.

### ETAT DE L'ART

Pour utiliser de manière efficace les insectes ou acariens auxiliaires destinés à la lutte biologique contre les ravageurs ou les plantes adventices, il est nécessaire de disposer d'un conditionnement permettant la protection de ces auxiliaires à la fois contre les prédateurs et contre l'environnement extérieur.

Les insectes ou acariens auxiliaires sont typiquement disposés dans un conditionnement à l'état d'adulte, à l'état larvaire, ou à l'état d'oeufs (parasités ou non), et peuvent être soit mobiles, soit immobiles.

Les auxiliaires sont préférentiellement choisis selon la période à laquelle ils éclosent, ou plus généralement selon la période ou date à laquelle ils seront aptes à sortir du conditionnement pour remplir leur fonction de prédateurs ou parasitoïdes des ravageurs et/ou antagonistes des plantes adventices. Il est ainsi possible de déterminer la date à laquelle un conditionnement comprenant des auxiliaires doit être utilisé, et disposé dans le milieu souhaité.

Jusqu'à présent, les conditionnements pour auxiliaires, sont présentés sous deux formes principales différentes :
- sous la forme de diffuseurs en carton en forme de plaquettes, qui s'accrochent directement sur les plantes, et qui sont donc utilisées manuellement ;
- sous la forme de capsules, plus ou moins sphériques, faites en carton, en cellulose moulée ou encore en plastique biodégradable, et qui peuvent être utilisées manuellement ou en épandage mécanisé, par moyens terrestres ou aériens.

L'épandage au sol de tels conditionnements présente un fort risque de mortalité des auxiliaires, notamment sur des cultures non totalement couvrantes où ces conditionnements peuvent se retrouver exposés directement au soleil, et ce d'autant plus que les cultures sont de petite taille.

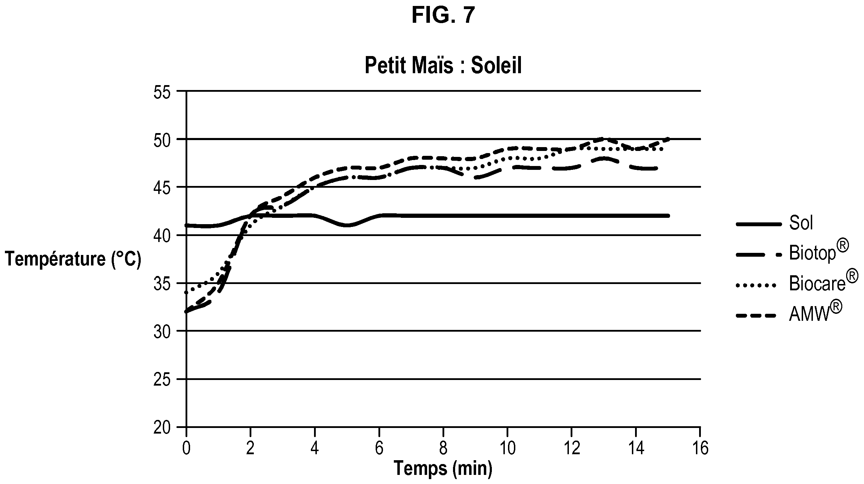

En effet, la température des conditionnements exposés au soleil peut dépasser rapidement la température létale des auxiliaires (pour les trichogrammes environ 38°C) ou provoquer une diminution drastique de leur qualité, en raison des phénomènes de conduction, convection et rayonnement. Comme visible sur la figure 7, qui est un diagramme représentant la température en fonction du temps pour trois conditionnements de type capsules de marques différentes (Biotop®, Biocare®, AMW®) qui ont été disposés dans un champ de petit maïs ainsi que la température du sol dudit champ, on peut en effet observer que la température des conditionnements dépasse la température du sol et peut atteindre 45°C en moins de 20 minutes après la pose, tuant ainsi les auxiliaires très rapidement. On peut également remarquer que la température des conditionnements est relativement stable après qu'elle ait atteint son maximum (qui est supérieur à 45°C), ainsi si les conditions météorologiques extérieures ne changent pas (température de l'air, exposition au soleil, etc...) la température des conditionnements est amenée à rester à de tels niveaux de température durant des heures. De plus cette exposition aux fortes températures peut se répéter sur plusieurs jours. Ainsi, aucun des trois modèles de conditionnements testés ne permet de garder une température acceptable.

D'autre part, les conditionnements au sol sont bien plus vulnérables à l'eau due aux pluies et/ou à l'irrigation. Cette eau va entraîner une humidification des conditionnements qui, si elle dure plusieurs jours, va provoquer le développement de moisissures dans les conditionnements, entrainant ainsi la mort des auxiliaires présents dans les conditionnements ou les empêchant d'en sortir.

Enfin, les conditionnements sont également vulnérables aux projections de boue consécutives à des pluies ou à l'irrigation qui sont susceptibles d'obstruer les ouvertures des conditionnements par lesquelles les auxiliaires sortent desdits conditionnements, piégeant ainsi les auxiliaires à l'intérieur desdits conditionnements et les empêchant de lutter contre les espèces nuisibles désirées.

Les attaques d'arthropodes prédateurs empêchent également les épandages au sol de conditionnements remplis d'auxiliaires d'avoir l'effet escompté. En effet, les prédateurs des auxiliaires, comme les fourmis, peuvent rentrer dans les conditionnements et dévorer les auxiliaires. Une solution connue pour limiter les attaques de prédateurs est de limiter la taille des ouvertures dans les conditionnements par lesquelles les auxiliaires sortent desdits conditionnements à de très faibles diamètres, de sorte que les prédateurs ne puissent pas rentrer dans les conditionnements tout en permettant aux auxiliaires de sortir. Cependant, une telle solution pose des problèmes de forte probabilité d'obstruction des ouvertures par la boue à cause de leurs très faibles diamètres.

Ainsi, les techniques connues pour l'épandage directement au sol de conditionnements d'auxiliaires dans des cultures n'apportent pas satisfaction. D'autres conditionnements pour auxiliaires sont connus des documents EP 2 380 437, US 2005/0120963 et US 2 403 840.

### PRÉSENTATION GÉNÉRALE DE L'INVENTION

Pour pouvoir épandre au sol les conditionnements dans les cultures et assurer une bonne émergence des auxiliaires, il est donc primordial de protéger les conditionnements des facteurs de mortalités des auxiliaires que sont la chaleur, les intempéries et les prédateurs.

En particulier, selon un aspect, l'invention consiste en un ensemble comprenant un conditionnement d'auxiliaires pour la lutte contre les ravageurs des cultures ou les plantes adventices et un étui pour la protection de ce conditionnement, l'ensemble étant caractérisé en ce que l'étui comprend une paroi protectrice adaptée pour entourer le conditionnement de manière à protéger le conditionnement contre la chaleur, l'eau et la boue, la paroi protectrice comprenant une ouverture adaptée pour permettre aux auxiliaires de sortir de l'étui.

Selon l'invention, la paroi protectrice est mobile entre une position repliée dans laquelle la paroi protectrice est plaquée contre le conditionnement, et une position déployée dans laquelle la paroi protectrice s'étend au moins en partie à distance du conditionnement, de sorte à créer un espace entre la paroi protectrice et le conditionnement autorisant une circulation d'air entre la paroi protectrice et le conditionnement.

Selon une caractéristique additionnelle, la paroi protectrice est à mémoire de forme, de sorte que lorsque la paroi protectrice est en position repliée elle tend à revenir spontanément en position déployée.

Selon une autre caractéristique, la paroi protectrice est adaptée pour qu'une ouverture du conditionnement permettant aux auxiliaires de sortir du conditionnement s'étende au niveau d'une ligne de contact entre le conditionnement et la paroi protectrice, lorsque le conditionnement est disposé à l'intérieur de la paroi protectrice et que la paroi protectrice est en position déployée.

Selon une caractéristique supplémentaire, l'étui présente une forme plane lorsque la paroi protectrice est dans une position repliée.

Selon une caractéristique particulière, la paroi protectrice comprend un dispositif de maintien du conditionnement pour bloquer le conditionnement à l'intérieur de l'étui.

Selon une caractéristique additionnelle, le dispositif de maintien du conditionnement comprend au moins une butée empêchant le conditionnement de sortir de l'étui par l'ouverture de la paroi protectrice.

Selon une autre caractéristique, la paroi protectrice comprend deux ouvertures, et le dispositif de maintien du conditionnement comprend deux butées situées respectivement à proximité des ouvertures.

Selon une caractéristique supplémentaire, le conditionnement est un diffuseur en plaquette ayant une longueur, et la paroi protectrice a une longueur supérieure à la longueur du conditionnement.

Selon une caractéristique particulière, la paroi protectrice est réalisée en carton ou en plastique biodégradable, éventuellement revêtue d'un vernis ou d'un répulsif contre des prédateurs des auxiliaires.

Selon une caractéristique additionnelle, l'étui est un tube de section constante selon un axe central, le conditionnement est un diffuseur en plaquette comprenant des cannelures qui forment des ouvertures permettant aux auxiliaires de sortir du conditionnement et qui sont dirigées perpendiculairement par rapport à l'axe central lorsque le conditionnement est à l'intérieur de l'étui.

Selon un aspect additionnel, l'invention consiste en un procédé d'épandage d'au moins un conditionnement d'auxiliaires pour la protection des cultures, caractérisé en ce qu'il comprend une étape consistant à disposer l'ensemble selon l'une des caractéristiques précédemment décrite dans les cultures à protéger.

Selon une caractéristique supplémentaire, le procédé comprend les étapes suivantes :
- stocker l'ensemble, la paroi protectrice étant maintenue en position repliée ;
- disposer l'ensemble dans les cultures à protéger, la paroi protectrice des étuis étant en position déployée.

Selon une caractéristique particulière, l'étape de stockage est réalisée dans un chargeur d'une machine d'épandage dans lequel une pluralité d'ensembles sont empilés les uns contre les autres en position repliée, et l'étape de disposition est réalisée en distribuant chacun des ensembles avec la machine d'épandage dans les cultures à protéger, la paroi protectrice des étuis adoptant spontanément la position déployée lorsque les ensembles sont distribués.

L'épandage de tels ensembles peut être aussi bien mécanisé à partir de vecteurs terrestres ou aériens, que complètement manuel.

### DESCRIPTIF DES FIGURES

D'autres caractéristiques, buts et avantages de la présente invention apparaitront à la lecture de la description détaillée qui va suivre, et en regard des dessins annexés, donnés à titre d'exemples non limitatifs et sur lesquels :
- la figure 1 représente une vue en perspective d'un étui selon un premier mode de réalisation, l'étui étant en position déployée ;
- la figure 2 représente la même vue que la figure 1, mais à l'intérieur un conditionnement du type diffuseur en plaquette contenant les auxiliaires est placé à l'intérieur de l'étui ;
- la figure 3 représente la même vue que la figure 2, mais les parties cachées du conditionnement sont représentées par des pointillés ;
- la figure 4 représente une vue de face de l'étui selon le premier mode de réalisation qui est en position repliée et à l'intérieur duquel est placé un conditionnement ;
- la figure 5 représente la même vue que la figure 4, l'étui étant dans une position intermédiaire entre la position repliée et la position déployée ;
- la figure 6 représente une vue en perspective du conditionnement représenté dans les figures 1 à 5 ;
- la figure 7 représente un diagramme de la température en fonction du temps pour trois modèles de conditionnements existants qui ont été disposé dans un champ de petit maïs ainsi que la température du sol dudit champ (température de l'air sous abri de 31.5°C) ;
- la figure 8 représente un diagramme de la température en fonction du temps pour un conditionnement seul et un conditionnement avec un étui qui ont été disposés dans un champ en plein soleil, ainsi que la température de l'air au voisinage du sol.

### DESCRIPTION D'UN OU PLUSIEURS MODES DE REALISATION DETAILLES

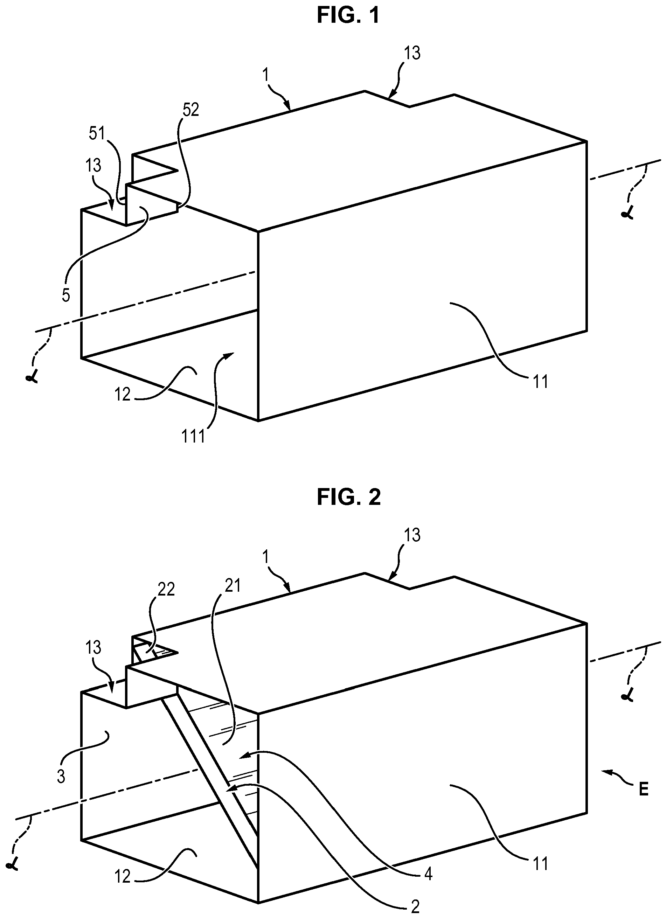

Comme représenté dans la figure 1, un étui 1 selon un premier mode de réalisation est un tube de section constante avec un axe central α et comprend une paroi protectrice 11 qui délimite une cavité 111.

La paroi protectrice 11 est une feuille plane qui est pliée successivement selon quatre lignes de pliage qui sont parallèles à l'axe central a, de sorte à former quatre arêtes qui délimitent quatre faces de l'étui 1. Les faces de l'étui 1 sont reliées deux à deux par les arêtes.

Dans le premier mode de réalisation, l'étui 1 est un tube à section constante en forme de losange. On entend ici par section, une section perpendiculaire à l'axe central a.

Le pliage de la paroi protectrice 11 délimite ainsi deux ouvertures 12 en losange disposées de part et d'autre de la cavité 111. Les deux ouvertures 12 correspondent à des surfaces non couvertes par la paroi protectrice 11.

Comme visible dans les figures 2 à 5 (où une seule ouverture 12 est visible), les ouvertures 12 de la paroi protectrice 11 permettent à un utilisateur de disposer un conditionnement 2 dans la cavité 111 de sorte que la paroi protectrice 11 entoure le conditionnement 2. Lorsqu'un conditionnement 2 est placé à l'intérieur d'un étui 1, ces deux éléments forment un ensemble E.

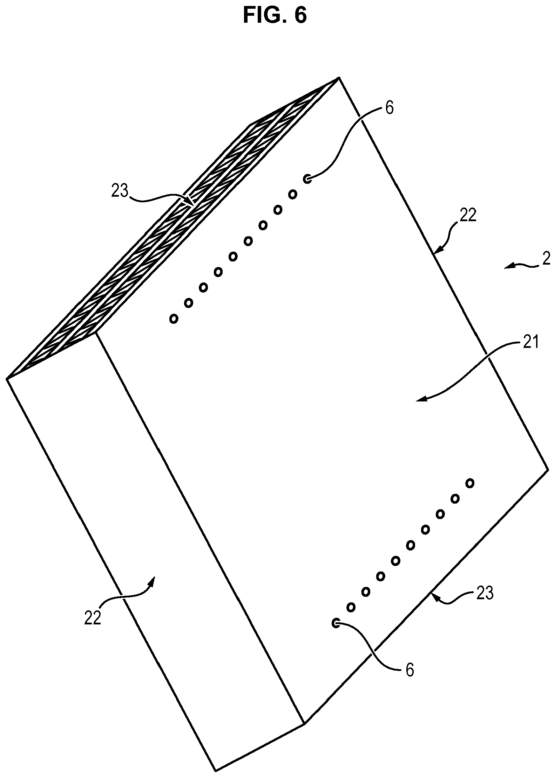

Dans le premier mode de réalisation, le conditionnement 2 est un diffuseur en plaquette plane formant un parallélépipède rectangle, comme représenté dans la figure 6, et qui comprend à titre d'exemple non limitatif: une longueur de 5,5 cm, une largeur de 4.5 cm, et une épaisseur de 0.3 cm. Le conditionnement 2 comprend deux faces principales 21, deux faces latérales 22, et deux faces de sortie 23. Par exemple, le conditionnement 2 peut être un diffuseur tel que décrit dans la demande de brevet WO2011/131513.

Le conditionnement 2 comprend des ouvertures afin que les trichogrammes disposés dans une cavité interne du conditionnement 2 puissent sortir dudit conditionnement 2 par lesdites ouvertures. Comme indiqué dans la demande de brevet WO2011/131513, les ouvertures peuvent être réalisées par des cannelures du carton. Dans le premier mode de réalisation, les cannelures définissant les ouvertures sont dirigées selon la largeur du conditionnement 2, et lesdites ouvertures sont disposées sur les faces de sorties 23. Lorsque le conditionnement 2 est placé à l'intérieur de l'étui 1, les cannelures sont perpendiculaires à l'axe central α et sont dirigées selon la diagonale de la section carrée de l'étui 1. Comme représenté dans la figure 6, le conditionnement 2 comprend d'une manière optionnelle des petits trous d'aiguilles 6 (préférentiellement d'un diamètre ne laissant pas passer les prédateurs des auxiliaires) réalisés sur les 2 faces principales 21 afin de permettre aux auxiliaires de sortir du conditionnement 2 par les faces principales 21.

Le conditionnement 2 peut aussi être un diffuseur en plaquette réalisé par un empilement de plusieurs couches de cartons non cannelé qui comprend des canaux de sortie périphériques disposés sur les faces de sorties 23 et qui permettent aux auxiliaires de sortir dudit conditionnement 2.

D'une manière additionnelle, le conditionnement 2 peut également être un diffuseur en plaquette ne présentant pas de trous périphériques mais uniquement des petits trous d'aiguilles 6 (préférentiellement d'un diamètre ne laissant pas passer les prédateurs des auxiliaires) réalisés sur les 2 faces principales 21.

Selon une variante possible, les deux faces principales 21 du conditionnement 2 sont renforcées au niveau des trous 6 afin d'empêcher d'éventuels prédateurs (comme les fourmis) d'agrandir les trous 6 en déchirant le conditionnement 2 au niveau desdits trous 6. Le renforcement peut être réalisé uniquement au niveau des trous 6, ou bien être réalisé sur l'intégralité des faces principales 21. Les faces principales 21 peuvent être renforcées en appliquant un vernis, en réalisant une pellicule protectrice (un pelliculage) avec un film plastique (par exemple en polymère biodégradable), ou en utilisant une plaque en un matériau plastique (par exemple en un polymère biodégradable) pour former les faces principales 21. Le conditionnement 2 peut donc être formé d'une plaquette creuse constitué en un matériau plastique (de préférence biodégradable). Afin de permettre aux auxiliaires de sortir du conditionnement, une fois les parois principales 21 recouvertes du vernis ou du pelliculage, les trous 6 sont réalisés dans lesdites parois principales 21, par exemple avec une aiguille de diamètre déterminé. Si les parois principales 21 sont formées par les plaques en un matériau plastique, alors les trous 6 sont réalisés directement dans lesdites plaques en un matériau plastique. Selon une variante possible, la couche de vernis ou le pelliculage recouvrent intégralement les faces principales 21. Selon une autre variante possible, seule la partie des faces principales 21 sur laquelle les trous 6 sont pratiqués est recouverte par la couche de vernis ou le pelliculage.

Les trous 6 peuvent être situés sur la partie des faces principales 21 qui est la plus éloignées des ouvertures 12 lorsque le conditionnement 2 est placé à l'intérieur d'un étui 1. Pour ce faire, les trous 6 peuvent être réalisés de telle sorte qu'ils forment une ligne qui est perpendiculaire aux deux faces de sortie 23 (et donc qui est parallèle aux deux faces latérales 22), la ligne étant située au milieu des faces principales 21. Ainsi, les éventuelles projections de boues qui peuvent survenir durant l'utilisation de l'ensemble E ont une probabilité plus faible de boucher les trous 6.

Selon une autre variante possible, le conditionnement 2 est enduit d'un produit répulsif contre les prédateurs des auxiliaires, le produit répulsif devant ne pas être toxique pour les auxiliaires puisqu'il est directement mis sur le conditionnement 2 qui contient lesdits auxiliaires.

Les dimensions de l'étui 1 sont les suivantes :
- l'étui 1 comprend une longueur suivant l'axe central α supérieure ou égale à la longueur du conditionnement 2 afin que la paroi protectrice 11 entoure le conditionnement 2 sur toute sa longueur ;
- la diagonale de la section en losange de l'étui 1 est légèrement supérieure, par exemple de 5mm, à la largeur du conditionnement 2 de sorte que lorsque le conditionnement 2 est installé à l'intérieur de l'étui 1, sa largeur soit dirigée dans la même direction que la diagonale de la section carrée de l'étui 1.

Dans le premier mode de réalisation, la paroi protectrice 11 comprend deux ouvertures 12. Selon une variante, l'étui 1 peut ne comprendre qu'une seule ouverture 12.

La paroi protectrice 11 de l'étui 1 permet de protéger le conditionnement 2 contre l'échauffement crée par le soleil, l'eau due à la pluie ou à l'irrigation, et contre les projections de boue.

La paroi protectrice 11 permet en effet d'agir sur le rayonnement direct du soleil en plaçant le conditionnement 2 à l'ombre, et donc en supprimant l'échauffement dû au rayonnement du soleil. Cette paroi protectrice 11 évite aussi l'échauffement du conditionnement 2 par conduction de la chaleur d'un sol chaud et par convection due à l'air s'échauffant au contact du sol.

La paroi protectrice 11 permet également de couvrir le conditionnement 2 et ainsi empêche la majorité de l'eau de pluie ou d'irrigation d'atteindre le conditionnement 2, empêchant une forte humidification de celui-ci et réduisant ainsi le risque de formation de moisissures nuisibles aux auxiliaires à l'intérieur du conditionnement 2.

La paroi protectrice 11 permet aussi de couvrir les ouvertures réalisées dans le conditionnement 2 afin que les auxiliaires puissent sortir du conditionnement 2 et ainsi empêche d'éventuelles projection de boue d'obstruer ces ouvertures.

De plus, la paroi protectrice 11 permet de protéger le conditionnement 2 contre les attaques des prédateurs des auxiliaires, comme les fourmis. En effet, l'utilisation de l'étui 1 permet de réduire le diamètre des ouvertures par lesquelles les auxiliaires sortent du conditionnement 2 sans augmenter le risque de colmatage desdites ouvertures par la boue, car la paroi protectrice 11 protège le conditionnement 2 de la boue.

D'une manière additionnelle, afin d'augmenter la protection contre les prédateurs, il est possible d'enduire la paroi protectrice 11 de produits répulsifs. Une telle solution permet d'utiliser des produits répulsifs sans les mettre directement en contact avec les auxiliaires présents dans le conditionnement 2, alors que les prédateurs souhaitant rentrer dans le conditionnement 2 sont obligés de marcher sur la paroi protectrice 11, et rentrent donc directement en contact avec les produits répulsifs. On comprendra évidement qu'une telle solution demande d'utiliser des produits qui ne sont pas nuisibles pour les auxiliaires, de sorte que lorsque les auxiliaires sortent du conditionnement 2 et marchent sur la paroi protectrice 11, ils ne soient pas intoxiqués par les produits utilisés.

Cette protection contre les prédateurs par utilisation de répulsifs est avantageuse car la protection apportée par la limitation du diamètre des ouvertures du conditionnement 2 permettant aux auxiliaires de sortir dudit conditionnement 2 est limitée. En effet, la réduction du diamètre des ouvertures du conditionnement 2 est limitée par la taille des auxiliaires car le diamètre des ouvertures ne doit pas être inférieur au diamètre des auxiliaires afin de ne pas emprisonner les auxiliaires à l'intérieur du conditionnement 2. Ainsi, la solution de réduction du diamètre des ouvertures du conditionnement 2 est inefficace contre des prédateurs dont le diamètre est inférieur au diamètre des auxiliaires. L'utilisation de produits répulsifs sur l'étui 1 est donc une solution pour protéger les auxiliaires contre des prédateurs dont les techniques de protection connues ne sont pas efficaces.

Dans le premier mode de réalisation, qui est une variante de l'étui selon l'invention, la paroi protectrice 11 a une longueur supérieure à la longueur du conditionnement 2. Dans le cas où le conditionnement 2 est du type capsules, on comprendra que la paroi protectrice a une longueur supérieure au diamètre du conditionnement 2 ou de plusieurs conditionnements 2. Une telle caractéristique permet à l'étui 1 d'apporter une meilleure protection au conditionnement 2 contre la chaleur, l'humidité et la boue.

En effet, dans le cadre de la protection contre la chaleur, la paroi protectrice 11 ayant une longueur supérieure à la longueur du conditionnement 2, le conditionnement 2 est totalement couvert par la paroi protectrice 11 et les rayons du soleil ne peuvent plus atteindre le conditionnement 2. De plus, un tel étui permet également de protéger le conditionnement 2 contre l'échauffement par convection qui crée des courants d'air chaud montant du sol, car la paroi protectrice 11 limite fortement le contact entre ces courants d'air chaud montant du sol et le conditionnement 2. Enfin la paroi protectrice 11 empêche réchauffement par conduction car le conditionnement 2 n'est plus directement au contact du sol mais est isolé.

Dans le cadre de la protection contre l'eau et contre la boue, la paroi protectrice 11 ayant une longueur plus importante que la longueur du conditionnement 2, l'étui 1 protège plus efficacement le conditionnement 2 des projections d'eau dues à la pluie ou à l'irrigation, ainsi que des projections de boue en offrant une surface de protection plus importante.

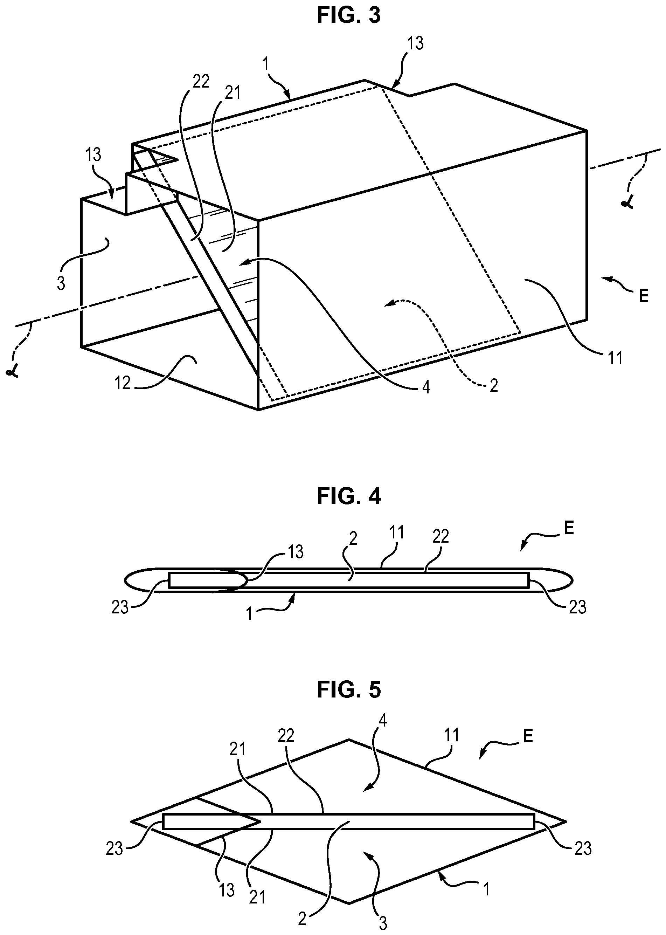

Selon le premier mode de réalisation, la paroi protectrice 11 de l'étui 1 est mobile entre une position déployée (illustrée par les figures 1 à 3) et une position repliée (illustrée par la figure 4). En position déployé, l'étui 1 comprend une longueur dirigée selon l'axe central a, une largeur et une épaisseur. La position déployée de la paroi protectrice 11 est telle que la paroi protectrice 11 s'étend en partie à distance du conditionnement 2. Plus précisément, la paroi protectrice 11 est en contact avec le conditionnement 2 uniquement suivant quatre lignes de contact au niveau des faces de sortie 23 du conditionnement 2. Encore plus précisément, la paroi protectrice 11 est en contact avec le conditionnement 2 sur les arêtes séparant les faces principales 21 d'avec les faces de sortie 23.

Lorsque la paroi protectrice est en position déployée, deux espaces 3 et 4 sont donc ainsi créés entre le conditionnement 2 et la paroi protectrice 11 au sein de la cavité 111. Plus précisément, les espaces 3 et 4 sont entre chacune des faces principales 21 et la paroi protectrice 11, les espaces 3 et 4 étant répartis de part et d'autre du conditionnement 2.

La position repliée de la paroi protectrice 11 est telle que la paroi protectrice 11 est plaquée contre le conditionnement 2 placé à l'intérieur de l'étui 1. Lorsque la paroi protectrice 11 est en position repliée, la paroi protectrice 11 est plaquée contre les faces principales 21 du conditionnement 2. Lorsque la paroi protectrice 11 est en position repliée, l'étui 1 présente une épaisseur très réduite égale à l'épaisseur du conditionnement 2 à laquelle s'ajoute l'épaisseur de la paroi protectrice 11. La longueur de l'étui 1 reste inchangée lorsque la paroi protectrice 11 est en position repliée, et en position repliée la largeur de l'étui 1 est supérieure à celle de l'étui 1 lorsque la paroi protectrice 11 est en position déployée (environ 25% de plus).

L'étui 1 est ainsi stocké avec la paroi protectrice 11 en position repliée, le conditionnement 2 étant placé à l'intérieur, afin de limiter l'encombrement du stock d'ensembles E. En effet, comme illustré par la figure 4, l'étui 1 présente une forme plane lorsque la paroi protectrice 11 est en position repliée, limitant ainsi fortement l'encombrement de l'étui 1 et donc de l'ensemble E.

Lorsque l'ensemble E est placé dans une culture, la paroi protectrice 11 de l'étui 1 est en position déployée. Comme décrit précédemment, lorsque la paroi protectrice 11 est en position déployée, le conditionnement 2 a une surface en contact avec la paroi protectrice 11 limitée. Ainsi, le transfert de chaleur entre l'étui 1 et le conditionnement 2 est fortement limité. En effet, l'étui 1 est chauffé d'une part directement par les rayons du soleil, et d'autre part par le sol qui lui-même est chauffé par le soleil. De plus, la présence des espaces 3 et 4 entre la paroi protectrice 11 et le conditionnement 2 permet d'obtenir une bonne ventilation à l'intérieur de l'étui 1. Cette ventilation permet d'augmenter la vitesse de séchage du conditionnement 2 dans le cas où le conditionnement 2 est humide, voir mouillé. Un séchage rapide permet de limiter fortement le risque de développement de moisissures à l'intérieur du conditionnement 2.

Un tel étui 1 selon le premier mode de réalisation permet d'améliorer encore plus la protection contre l'obstruction des ouvertures du conditionnement 2 par des projections de boue, car ces ouvertures réalisées sur les faces de sortie 23 du conditionnement 2 sont fortement protégées par la paroi protectrice 11. En effet, l'espace entre la paroi protectrice 11 et les ouvertures du conditionnement 2 est très faible. De plus, les ouvertures du conditionnement 2 sont entourées par les lignes de contact entre le conditionnement 2 et la paroi protectrice 11, ces lignes de contact empêchant les éventuelles projections de boue de venir encrasser les faces de sortie 23. Il en est de même pour les ouvertures en forme de trous d'aiguille qui seraient pratiquées sur les faces principales 21 des conditionnements, car elles seraient protégées par les parois 11.

D'une manière préférentielle, la paroi protectrice 11 est à mémoire de forme, de sorte qu'elle tende à revenir spontanément dans sa position déployée. Cette mémoire de forme peut être obtenue en utilisant un matériau élastique pour la réalisation de la paroi protectrice 11 (par exemple avec du carton à fibres longues), et/ou en utilisant des moyens élastiques, comme par exemple un ressort ou un élastique, qui tendent à ce que la paroi protectrice 11 prenne sa position déployée. La mémoire de forme de la paroi protectrice 11 peut également être améliorée en réalisant les lignes de pliage de la paroi protectrice 11 selon des techniques de pliage connues qui permettent de favoriser le retour spontané de la paroi protectrice 11 dans sa position déployée (par exemple mais non exclusivement à base de pré-pliages ménageant des bourrelets de matière).

Le fait que la paroi protectrice 11 soit à mémoire de forme permet de simplifier l'épandage des ensembles E dans les cultures. En effet, la mémoire de forme permet de projeter les ensembles E dans les cultures, par exemple avec une machine d'épandage ou à la main, la paroi protectrice 11 passant de la position repliée à la position déployée durant la projection. Ainsi, il est possible d'épandre facilement des ensembles E par des moyens aériens (avion, ULM, hélicoptère, drone...) et par moyens terrestres (tracteurs, quad, moto...) permettant ainsi de couvrir de très grandes superficies de culture en peu de temps.

Afin de maintenir le conditionnement 2 à l'intérieur de l'étui 1, la paroi protectrice 11 peut comprendre un dispositif de maintien en position du conditionnement dans la cavité 111. Dans le premier mode de réalisation exposé dans les figures 1 à 5, le dispositif de maintien est réalisé par des butées 13. Les butées 13 sont disposées au niveau de chacune des ouvertures 12, de sorte que lorsqu'un conditionnement 2 est placé à l'intérieur de l'étui 1, une butée 13 est disposée entre le conditionnement 2 et une ouverture 12, empêchant ainsi le conditionnement 2 de sortir par l'ouverture 12 en le bloquant à l'intérieur de l'étui 1.

Les deux butées 13 sont réalisées en découpant la paroi protectrice 11 à proximité des ouvertures 12, par exemple à une distance comprise entre 1cm et 2cm. Plus précisément on réalise deux découpes 5 sur une même arête, les découpes 5 étant perpendiculaires à l'arête. Les découpes 5 s'étendent sur les deux faces adjacentes à l'arête d'une façon symétrique par rapport à l'arête selon deux demi-découpes 51 et 52. Les deux parties de la paroi protectrice 11 qui ne sont pas situées entre les deux découpes 5 sont repliées vers l'intérieur de l'étui 1, formant ainsi les butées 13 en forme de L qui font saillie à l'intérieur de la cavité 111 et qui crée un obstacle au niveau de l'ouverture 12. A titre d'exemple, les demi-découpes 51 et 52 ont une longueur sensiblement égale à un tiers de la largeur d'une face de l'étui 1. Ainsi, les butées 13 forment chacune un L avec deux bras de longueur égale à un tiers de la largeur d'une face de l'étui 1. Les découpes 5 sont espacées entre elle d'une distance sensiblement égale à la longueur du conditionnement 2, de sorte que le conditionnement 2 puisse être installé entre les deux butées 13.

De telles butées 13 permettent de maintenir le conditionnement 2 à l'intérieur de l'étui 1 que la paroi protectrice soit en position repliée, déployée, ou dans une position intermédiaire entre repliée et déployée.

D'une façon préférentielle, les butées 13 sont escamotables afin qu'un utilisateur ou une machine puisse mettre en place un conditionnement 2 ou l'enlever de l'intérieur de l'étui 1 en rentrant les butées 13 puis en les remettant en place.

Lors de la mise en oeuvre d'un étui 1, une première butée 13 est repliée de sorte à faire saillie à l'intérieur de la cavité 111, puis on procède à l'introduction du conditionnement 2 par l'ouverture 12 opposée de l'étui 1, et enfin on replie la deuxième butée 13 pour immobiliser le conditionnement 2 à l'intérieur de l'étui 1.

Les butées 13 permettent également d'augmenter l'élasticité de la paroi protectrice 11, en jouant le rôle d'un ressort, améliorant ainsi sa caractéristique de mémoire de forme. En effet, de telles butées 13 tendent à faire placer la paroi protectrice 11 dans sa position déployée.

Le nombre de butées 13 n'est pas limité à deux, l'étui 1 peut comprendre un nombre supérieur de butées 13. De plus, les butées 13 peuvent être réalisées sur des arêtes différentes. Enfin, les dispositifs de maintien ne sont pas limités au système des butées 13 précédemment décrit, mais peut être constitué par n'importe quel moyen permettant de maintenir le conditionnement 2 à l'intérieur de l'étui 1. Ainsi, les dispositifs de maintien peuvent être constitués par des feuilles percées qui sont collées sur la paroi protectrice 11 de sorte à obstruer les ouvertures 12 pour empêcher le conditionnement 2 de sortir par les ouvertures 12, tout en permettant la ventilation de l'étui 1 et aux auxiliaires de sortir de l'étui 1.

La paroi protectrice 11 de l'étui 1 est préférentiellement fabriquée à partir de matière biodégradable. Ainsi, la paroi protectrice 11 peut être fabriquée en carton, en papier fort, ou en polymère biodégradable. La paroi protectrice 11 peut être recouverte d'un vernis protecteur afin de lui permettre d'être étanche et de résister au déchirement. De plus, de tels matériaux possèdent une bonne élasticité intrinsèque, permettant ainsi à la paroi protectrice d'être à mémoire de forme.

La paroi protectrice 11 de l'étui est préférentiellement de couleur blanche ou claire pour minimiser l'échauffement du au rayonnement solaire.

Dans le mode de réalisation présenté, le conditionnement 2 est un diffuseur en plaquette qui est de forme plane à faible épaisseur, mais l'étui 1 n'est pas limité à la protection de diffuseur en plaquette, mais peut être utilisé pour protéger tous types de conditionnements. Ainsi, pour protéger un conditionnement d'une forme différente, la forme de la paroi protectrice 11 peut être adaptée à la forme du conditionnement à protéger. Par exemple, la paroi protectrice 11 peut être en forme d'un tube de section constante cylindrique de révolution sans arête, ou bien d'un tube de section constante ovale comprenant deux arêtes.

Un procédé d'épandage dans une culture de plusieurs ensembles E comprend une étape consistant à disposer les ensembles E dans la culture à protéger.

L'étape de disposition peut être réalisée à la main, par exemple en déposant manuellement les ensembles E individuellement dans la culture, ou bien de manière automatique, par exemple en distribuant les ensembles E dans la culture avec une machine d'épandage.

Selon une variante préférentielle, les ensembles E sont d'abord stockés en étant empilés et comprimés les uns contre les autres dans un chargeur d'une machine d'épandage, les parois protectrices 11 étant en position repliée, ce qui permet de fortement réduire le volume occupé. Puis, les ensembles E sont distribués par la machine d'épandage dans la culture à protéger. Les parois protectrices 11 des étuis 1 des ensembles E étant à mémoire de forme, à la sortie de la machine et durant la distribution, les parois protectrices 11 adoptent spontanément leur position déployée et tombe sur le sol de la culture en position déployée.

La machine d'épandage peut être disposée dans un moyen aérien (par exemple un avion) et les ensembles E peuvent être projetés dans la culture depuis le moyen aérien, ce qui permet ainsi de couvrir une culture de grande superficie. Il est également possible d'utiliser un moyen terrestre.

Selon une variante, les ensembles E ne sont pas distribués dans la culture par une machine d'épandage, mais sont projetés manuellement.

Selon une variante qui ne fait pas partie de l'invention et qui est plus adaptée pour les cultures de faibles superficies, les ensembles E comprennent un étui 1 dont la paroi protectrice 11 est rigide et est limitée à une position déployée. Ces ensembles E sont ensuite posés à la main dans la culture à protéger. Dans cette variante, la paroi protectrice 11 est préférentiellement dans une matière plastique résistante qui permet de réutiliser l'étui 1 d'une fois sur l'autre en changeant le conditionnement 2 à chaque nouvelle utilisation.

Les ensembles E sont préférentiellement disposés dans les cultures de manière à reposer sur une face de l'étui 1, de sorte que les ouvertures 12 sont dirigées transversalement par rapport à la surface du sol des cultures. La forme allongée des ensembles les prédispose à se placer spontanément dans cette position d'équilibre stable.

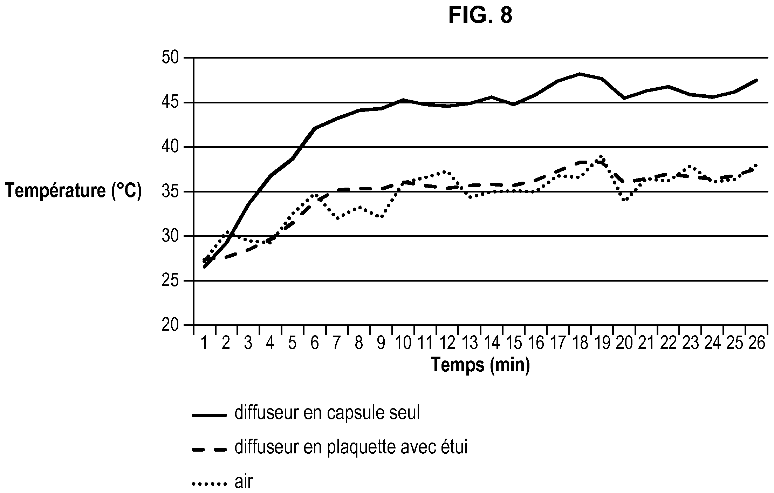

Comme présenté par le diagramme de la figure 8, qui présente l'évolution de la température pour un conditionnement en capsule seul et un conditionnement 2 en diffuseur en plaquette avec un étui 1 qui sont disposés dans une culture non couvrante par rapport à l'évolution de la température de l'air au voisinage du sol, l'invention permet de réduire sensiblement l'échauffement du conditionnement 2. En effet, avec un étui 1 conforme au mode de réalisation présenté dans les figures 1 à 6, la température du conditionnement 2 est maintenue à la même température que l'air alors qu'un conditionnement seul peut atteindre des températures de plus de 10°C supérieures à la température de l'air.

## Revendications

1. Ensemble (E) comprenant un conditionnement (2) d'auxiliaires pour la protection des cultures et un étui (1) pour la protection de ce conditionnement (2), l'étui (1) comprenant une paroi protectrice (11) adaptée pour entourer le conditionnement (2) de manière à protéger le conditionnement (2) contre la chaleur, l'eau et la boue, la paroi protectrice (11) comprenant une ouverture (12) adaptée pour permettre aux auxiliaires de sortir de l'étui (1), l'ensemble étant **caractérisé en ce que** la paroi protectrice (11) est mobile entre une position repliée dans laquelle la paroi protectrice (11) est plaquée contre le conditionnement (2) et une position déployée dans laquelle la paroi protectrice (11) s'étend au moins en partie à distance du conditionnement (2).

2. Ensemble (E) selon la revendication précédente, dans lequel la paroi protectrice (11) est à mémoire de forme, de sorte que lorsque la paroi protectrice (11) est en position repliée elle tend à revenir spontanément en position déployée.

3. Ensemble (E) selon l'une des revendications précédentes, dans lequel la paroi protectrice (11) est adaptée pour qu'une ouverture du conditionnement (2) permettant aux auxiliaires de sortir du conditionnement (2) s'étende au niveau d'une ligne de contact entre le conditionnement (2) et la paroi protectrice (11), lorsque le conditionnement (2) est disposé à l'intérieur de la paroi protectrice (11) et que la paroi protectrice (11) est en position déployée.

4. Ensemble (E) selon l'une des revendications 1 à 3, dans lequel l'étui (1) présente une forme plane lorsque la paroi protectrice (11) est dans une position repliée.

5. Ensemble (E) selon l'une des revendications précédentes, dans lequel la paroi protectrice (11) comprend un dispositif de maintien (13) du conditionnement (2) pour bloquer le conditionnement (2) à l'intérieur de l'étui (1).

6. Ensemble (E) selon la revendication précédente, dans lequel le dispositif de maintien (13) du conditionnement comprend au moins une butée (13) empêchant le conditionnement (2) de sortir de l'étui (1) par l'ouverture (12) de la paroi protectrice (11).

7. Ensemble (E) selon la revendication 5, dans lequel la paroi protectrice (11) comprend deux ouvertures (12), et dans lequel le dispositif de maintien (13) du conditionnement comprend deux butées (13) situées respectivement à proximité des ouvertures (12).

8. Ensemble (E) selon l'une des revendications précédentes, dans lequel le conditionnement (2) est un diffuseur en plaquette ayant une longueur, et la paroi protectrice (11) a une longueur supérieure à la longueur du conditionnement (2).

9. Ensemble (E) selon l'une des revendications précédentes, dans lequel la paroi protectrice (11) est réalisée en carton ou en plastique biodégradable, éventuellement revêtue d'un vernis ou d'un répulsif contre des prédateurs des auxiliaires.

10. Ensemble (E) selon l'une des revendications précédentes, dans lequel l'étui (1) est un tube de section constante selon un axe central (a), le conditionnement (2) est un diffuseur en plaquette comprenant des cannelures qui forment des ouvertures permettant aux auxiliaires de sortir du conditionnement 2 et qui sont dirigées perpendiculairement par rapport à l'axe central (α) lorsque le conditionnement (2) est à l'intérieur de l'étui (1).

11. Procédé d'épandage d'au moins un conditionnement (2) d'auxiliaires pour la protection des cultures, comprenant l'étape suivante :
- disposer un ensemble (E) dans les cultures à protéger, l'ensemble (E) comprenant un conditionnement (2) d'auxiliaires pour la protection des cultures et un étui (1) pour la protection de ce conditionnement (2), l'étui (1) comprenant
une paroi protectrice (11) adaptée pour entourer le conditionnement (2) de manière à protéger le conditionnement (2) contre la chaleur, l'eau et la boue, la paroi protectrice (11) comprenant une ouverture (12) adaptée pour permettre aux auxiliaires de sortir de l'étui (1), l'ensemble étant **caractérisé en ce que** la paroi protectrice (11) est mobile entre une position repliée dans laquelle la paroi protectrice (11) est plaquée contre le conditionnement (2) et une position déployée dans laquelle la paroi protectrice (11) s'étend au moins en partie à distance du conditionnement (2).

12. Procédé selon la revendication 11, dont l'ensemble (E) est conforme à la revendication 2, comprenant les étapes suivantes :
- stocker l'ensemble (E), la paroi protectrice (11) étant maintenue en position repliée ;
- disposer l'ensemble (E) dans les cultures à protéger, la paroi protectrice (11) des étuis (1) étant en position déployée.

13. Procédé selon la revendication 11, **caractérisé en ce que** l'étape de stockage est réalisée dans un chargeur d'une machine d'épandage dans lequel une pluralité d'ensembles (E) sont empilés les uns contre les autres en position repliée, et que l'étape de disposition est réalisée en distribuant chacun des ensembles (E) avec la machine d'épandage dans les cultures à protéger, la paroi protectrice (11) des étuis (1) adoptant spontanément la position déployée lorsque les ensembles (E) sont distribués.

## Patentansprüche

1. Einheit (E), eine Verpackung (2) für Organismen für den Schutz der Kulturen und eine Hülle (1) für den Schutz dieser Verpackung (2) umfassend, wobei die Hülle (1) eine Schutzwand (11) umfasst, die ausgeführt ist, um die Verpackung (2) zu umgeben, um die Verpackung (2) gegen die Hitze, das Wasser und Schlamm zu schützen, wobei die Schutzwand (11) eine Öffnung (12) umfasst, die ausgeführt ist, um es den Organismen zu ermöglichen, aus der Hülle (1) herauszutreten, wobei die Einheit **dadurch gekennzeichnet ist, dass** die Schutzwand (11) zwischen einer eingefalteten Position, in der die Schutzwand (11) gegen die Verpackung (2) gepresst wird, und einer auseinandergefalteten Position beweglich ist, in der sich die Schutzwand (11) mindestens teilweise auf Abstand zu der Verpackung (2) erstreckt.

2. Einheit (E) nach dem vorstehenden Anspruch, wobei die Schutzwand (11) mit einem Formgedächtnis ist, sodass, wenn die Schutzwand (11) in der eingefalteten Position ist, sie dahin tendiert, spontan in die auseinandergefaltete Position zurückzukehren.

3. Einheit (E) nach einem der vorstehenden Ansprüche, wobei die Schutzwand (11) ausgeführt ist, damit sich eine Öffnung der Verpackung (2), die es den Organismen ermöglicht, aus der Verpackung (2) herauszutreten, auf Höhe einer Berührungslinie zwischen der Verpackung (2) und der Schutzwand (11) erstreckt, wenn die Verpackung (2) im Inneren der Schutzwand (11) angeordnet ist und die Schutzwand (11) in der auseinandergefalteten Position ist.

4. Einheit (E) nach einem der Ansprüche 1 bis 3, wobei die Hülle (1) eine ebene Form darbietet, wenn die Schutzwand (11) in einer eingefalteten Position ist.

5. Einheit (E) nach einem der vorstehenden Ansprüche, wobei die Schutzwand (11) eine Haltevorrichtung (13) der Verpackung (2) umfasst, um die Verpackung (2) im Inneren der Hülle (1) zu blockieren.

6. Einheit (E) nach dem vorstehenden Anspruch, wobei die Haltevorrichtung (13) der Verpackung mindestens einen Anschlag (13) umfasst, der die Verpackung (2) daran hindert, aus der Hülle (1) durch die Öffnung (12) der Schutzwand (11) herauszutreten.

7. Einheit (E) nach Anspruch 5, wobei die Schutzwand (11) zwei Öffnungen (12) umfasst, und wobei die Haltevorrichtung (13) der Verpackung zwei Anschläge (13) umfasst, die sich jeweils in der Nähe der Öffnungen (12) befinden.

8. Einheit (E) nach einem der vorstehenden Ansprüche, wobei die Verpackung (2) ein Plättchen-Diffusor ist, der eine Länge aufweist, und die Schutzwand (11) eine Länge aufweist, die größer ist, als die Länge der Verpackung (2).

9. Einheit (E) nach einem der vorstehenden Ansprüche, wobei die Schutzwand (11) aus Karton oder aus biologisch abbaubarem Kunststoff realisiert ist, eventuell mit einem Lack oder einem Abwehrmittel gegen Schmarotzer der Organismen beschichtet.

10. Einheit (E) nach einem der vorstehenden Ansprüche, wobei die Hülle (1) ein Rohr mit einem konstanten Querschnitt entlang einer Mittenachse (α) ist, die Verpackung (2) ein Plättchen-Diffusor ist, der Rillen umfasst, die Öffnungen bilden, die es den Organismen ermöglichen, aus der Verpackung (2) herauszutreten, und die senkrecht im Verhältnis zu der Mittenachse (α) geleitet werden, wenn die Verpackung (2) im Inneren der Hülle (1) ist.

11. Verfahren zum Ausbringen mindestens einer Verpackung (2) von Organismen für den Schutz von Kulturen, den folgenden Schritt umfassend:
- Anordnen einer Einheit (E) in den zu schützenden Kulturen, wobei die Einheit (E) eine Verpackung (2) für Organismen für den Schutz der Kulturen und eine Hülle (1) für den Schutz dieser Verpackung (2) umfasst, wobei die Hülle (1) umfasst
eine Schutzwand (11), die ausgeführt ist, um die Verpackung (2) zu umgeben, um die Verpackung (2) gegen die Hitze, das Wasser und Schlamm zu schützen, wobei die Schutzwand (11) eine Öffnung (12) umfasst, die ausgeführt ist, um es den Organismen zu ermöglichen, aus der Hülle (1) herauszutreten, wobei die Einheit **dadurch gekennzeichnet ist, dass** die Schutzwand (11) zwischen einer eingefalteten Position, in der die Schutzwand (11) gegen die Verpackung (2) gepresst wird, und einer auseinandergefalteten Position beweglich ist, in der sich die Schutzwand (11) mindestens teilweise auf Abstand zu der Verpackung (2) erstreckt.

12. Verfahren nach Anspruch 11, mit dessen Einheit (E) nach Anspruch 2, die folgenden Schritte umfassend:
- Lagern der Einheit (E), wobei die Schutzwand (11) in eingefalteter Position gehalten wird;
- Anordnen der Einheit (E) in den zu schützenden Kulturen, wobei die Schutzwand (11) der Hüllen (1) in auseinandergefalteter Position ist.

13. Verfahren nach Anspruch 11, **dadurch gekennzeichnet, dass** der Lagerungsschritt in einem Lader einer Ausbringungsmaschine realisiert wird, in der eine Vielzahl von Einheiten (E) in eingefalteter Position aneinander gestapelt sind, und dass der Anordnungsschritt durch Verteilen jeder der Einheiten (E) mit der Ausbringungsmaschine in den zu schützenden Kulturen realisiert wird, wobei die Schutzwand (11) der Hüllen (1), spontan die auseinandergefaltete Position einnimmt, wenn die Einheiten (E) verteilt werden.

## Claims

1. Assembly (E) comprising packaging (2) for biocontrol agents for protecting cultivations and a case (1) for protecting this packaging (2), the case (1) comprising a protective wall (11) suitable for surrounding the packaging (2) so as to protect the packaging (2) against heat, water and mud, the protective wall (11) comprising an opening (12) suitable for enabling the biocontrol agents to emerge from the case (1), the assembly being **characterised in that** the protective wall (11) is able to move between a folded position in which the protective wall (11) is pressed against the packaging (2) and a deployed position in which the protective wall (11) extends at least partly at a distance from the packaging (2).

2. Assembly (E) according to the preceding claim, wherein the protective wall (11) is of the shape-memory type, so that, when the protective wall (11) is in the folded position, it tends to return spontaneously to the deployed position.

3. Assembly (E) according to any of the preceding claims, wherein the protective wall (11) is suitable for an opening of the packaging (2) enabling the biocontrol agents to emerge from the packaging (2) to extend at a contact line between the packaging (2) and the protective wall (11), when the packaging (2) is disposed inside the protective wall (11) and that the protective wall (11) is in the deployed position.

4. Assembly (E) according to any of claims 1 to 3, wherein the case (1) has a planar shape when the protective wall (11) is in a folded position.

5. Assembly (E) according to any of the preceding claims, wherein the protective wall (11) comprises a device for holding (13) the packaging (2) in order to lock the packaging (2) inside the case (1).

6. Assembly (E) according to the preceding claim, wherein the device for holding (13) the packaging comprises at least one stop (13) preventing the packaging (2) from emerging from the case (1) through the opening (12) of the protective wall (11).

7. Assembly (E) according to claim 5, wherein the protective wall (11) comprises two openings (12), and wherein the device for holding (13) the packaging comprises two stops (13) situated respectively in the vicinity of the openings (12).

8. Assembly (E) according to any of the preceding claims, wherein the packaging (2) is a plate diffuser having a length, and the protective wall (11) has a length greater than the length of the packaging (2).

9. Assembly (E) according to any of the preceding claims, wherein the protective wall (11) is produced from cardboard or biodegradable plastics material, optionally covered with a varnish or a repellent against predators of the biocontrol agents.

10. Assembly (E) according to any of the preceding claims, wherein the case (1) is a tube with a constant cross-section along a central axis (α), the packaging (2) is a plate diffuser comprising grooves that form openings enabling the biocontrol agents to emerge from the packaging (2) and which are directed perpendicularly with respect to the central axis (α) when the packaging (2) is inside the case (1).

11. Method for spreading at least one packaging (2) for biocontrol agents for protecting cultivations, comprising the following step:
- disposing an assembly (E) in the cultivations to be protected, the assembly (E) comprising a packaging (2) for biocontrol agents for protecting cultivations and a case (1) for protecting this packaging (2), the case (1) comprising
a protective wall (11) suitable for surrounding the packaging (2) so as to protect the packaging (2) against heat, water and mud, the protective wall (11) comprising an opening (12) suitable for enabling the biocontrol agents to emerge from the case (1), the assembly being **characterised in that** the protective wall (11) is able to move between a folded position in which the protective wall (11) is pressed against the packaging (2) and a deployed position in which the protective wall (11) extends at least partly at a distance from the packaging (2).

12. Method according to claim 11, wherein the assembly (E) is in accordance with claim 2, comprising the following steps:
- storing the assembly (E), the protective wall (11) being held in the folded position;
- disposing the assembly (E) in the cultivations to be protected, the protective wall (11) of the cases (1) being in the deployed position.

13. Method according to claim 11, **characterised in that** the storage step is carried out in a magazine of a spreading machine in which a plurality of assemblies (E) are stacked against each other in the folded position, and that the disposition step is performed by distributing each of the assemblies (E) with the spreading machine in the cultivations to be protected, the protective wall (11) of the cases (1) spontaneously adopting the deployed position when the assemblies (E) are distributed.
